# EUROPÄISCHE PATENTSCHRIFT

(11) **EP 1 065 979 B1**
(45) Veröffentlichungstag und Bekanntmachung des Hinweises auf die Patenterteilung: **22.12.2004**
(21) Anmeldenummer: 99914548.5
(22) Anmeldetag: 26.03.1999
(51) Int. Cl.: A61B 17/28, A61B 17/32

(54) **CHIRURGISCHES INSTRUMENT MIT EINEM DURCHGEHENDEN HOHLKANAL FÜR EIN WEITERES INSTRUMENT**
SURGICAL INSTRUMENT WITH A CONTINUOUS HOLLOW CHANNEL FOR ANOTHER INSTRUMENT
INSTRUMENT CHIRURGICAL MUNI D'UN CANAL CREUX CONTINU POUR UN AUTRE INSTRUMENT

(30) Priorität: 27.03.1998 DE 19813781
(43) Veröffentlichungstag der Anmeldung: 10.01.2001
(73) Patentinhaber: Karl Storz GmbH & Co. KG, 78532 Tuttlingen (DE)
(72) Erfinder: WALLER, Peter, D-82131 Gauting (DE); MATERN, Ulrich, D-79283 Bollschweil (DE)
(74) Vertreter: Weller, Wolfgang, Dr.
(86) Internationale Anmeldenummer: PCT/EP1999/002080
(87) Internationale Veröffentlichungsnummer: WO 1999/049794

(56) Entgegenhaltungen:
- EP-A- 0 065 054
- EP-A- 0 279 358
- WO-A-95/08946
- WO-A-96/22056
- DE-A- 3 716 764
- DE-A- 19 632 298
- FR-A- 2 479 680
- US-A- 4 770 174
- US-A- 5 569 299

## Beschreibung

Die Erfindung betrifft ein chirurgisches Instrument gemäß der Präambel von Anspruch 1.

Ein derartiges chirurgisches Instrument ist aus der FR-A-2 479 680 bekannt.

Bei diesem chirurgischen Instrument kann bei gespreizten Arbeitselementen ein weiteres Instrument durch das Innere des chirurgischen Instrumentes hindurchgeführt werden, dessen Außendurchmesser unwesentlich geringer als der Durchmesser des Innenraums des Instrumentes ist.

Sind die Arbeitselemente geschlossen, kann das weitere eingeschobene Instrument nicht über das distale Ende des Schaftes hinausgeschoben werden, da die geschlossenen Arbeitselemente dies sperren.

Durch die zwischenzeitlich weit verbreitete minimal-invasive Chirurgie ist es üblich geworden, die im Körper stattfindenden Arbeitsvorgänge der chirurgischen Instrumente über Endoskope zu beobachten.

Diese Instrumente werden über Trokare in den Körper eingeführt, wobei zum ausgänglichen Einführen der Trokare in den Körper relativ kleine Inzisionen, also Einschnitte notwendig sind.

Wird ein chirurgisches Instrument, bspw. eine chirurgische Schere an einer anderen Stelle eingeführt als ein Endoskop, das den Schneidevorgang beobachten soll, sind zwei Inzisionen notwendig.

Da ein Bestreben in dieser Operationstechnik darin besteht, möglichst wenig Inzisionen anzufertigen, ist es bekannt geworden, mehrere Instrumente über eine Inzision in den Körper einzuführen.

Es ist Aufgabe der vorliegenden Erfindung, einen bis zum äußersten distalen Ende des Instrumentes reichenden Hohlkanal mit größtmöglichem Querschnitt zu ermöglichen.

Erfindungsgemäß wird die Aufgabe durch die Merkmale von Anspruch 1 gelöst

Diese Maßnahmen haben den Vorteil, daß etwa der gesamte innere Hohlraum des rohrförmigen Schaftes dazu zur Verfügung steht, um ein weiteres Instrument einzuführen. Der zur Verfügung stehende Querschnitt wird allenfalls noch durch das üblicherweise stangenförmige Betätigungselement eingeengt, falls dieses im Innern des Hohlraumes verläuft. Durch die Ausgestaltung der Arbeitselemente derart, daß diese weder im geschlossenen noch im gespreizten Zustand in den Querschnitt des Hohlraumes hineinreichen, steht dieser in jedem Betriebszustand bzw. Arbeitszustand der Arbeitselemente in vollem Querschnitt zur Verfügung. So ist es bspw. möglich, in den durchgehenden Hohlraum ein Endoskop einzuführen, über das die Manipulationen, die mit den Arbeitselementen durchgeführt werden sollen, visuell beobachtet werden können. Dadurch, daß die Arbeitselemente nicht in den Querschnitt des durchgehenden Hohlraumes hineinreichen, kann andauernd, bspw. beim Einführen des Instruments mit geschlossenen Maulteilen, dieser Vorgang durch das mittig eingeführte Endoskop beobachtet werden und dann, nach Einführen in den Körper, auch die Manipulationen der Arbeitselemente. Je nachdem, wie die Arbeitselemente ausgebildet sind, bzw. zu welchem Zweck sie vorgesehen sind, können diese zum Fassen und/oder zum Schneiden, zum Spreizen oder dgl. dienen. Da der gesamte Querschnitt des rohrförmigen Schaftes als Hohlraum zur Verfügung steht, kann bei relativ durchmessergeringen chirurgischen Instrumenten noch ein Endoskop mit ausreichender Licht- und Sichtstärke eingeschoben werden. Der Hohlkanal kann auch als Saug- und/oder Spülkanal herangezogen werden. Es ist auch die Kombination Saug-/Spülkanal und eingeführtes Endoskop möglich, wenn nämlich der Außendurchmesser des Endoskopschaftes geringer ist als der lichte Innendurchmesser des Hohlkanals, so daß in diesem Zwischenraum um das Endoskop herum noch Flüssigkeiten oder gasförmige Medien zu- bzw. abgeführt werden können. Die Arbeitselemente können auch als Spreizelemente bei einer Dissektion dienen, durch den Hohlraum können dann anderweitige Faß- oder Schneidinstrumente hindurchgeschoben werden.

Der Zusammenbau der ineinandergeschobenen Instrumente ist äußerst schlank, und als besonderer Vorteil ist hervorzuheben, daß die Funktionsfähigkeit des einen Instrumentes nicht durch das andere Instrument beeinträchtigt wird.

In einer weiteren Ausgestaltung der Erfindung erstrecken sich die am distalen Ende angeordneten Arbeitselemente in einer Arbeitsstellung etwa in einer Verlängerung der Rohrwand des rohrförmigen Schaftes.

Diese Maßnahme hat den Vorteil, daß in dieser Stellung das chirurgische Instrument im Bereich des Schaftes und der Arbeitselemente die Form eines Rohres inne hat, das zum Einführen in den Körper durch einen Trokar hindurchgeschoben werden kann, so daß eine besonders raumsparende Ausgestaltung geschaffen ist.

In einer weiteren Ausgestaltung der Erfindung sind die Arbeitselemente selbst rohr- bzw. rohrabschnittförmig.

Diese Maßnahme hat den Vorteil, daß die Arbeitselemente selbst einen großen Querschnitt aufweisen, aber seitlich nicht über den Schaft hinausstehen und den inneren Hohlkanal in einer maximalen Breite freigeben, so daß bei einer schlanken Bauweise des chirurgischen Instruments und maximaler Stabilität der Arbeitselemente ein maximaler innerer Hohlkanal zur Verfügung steht.

In einer weiteren Ausgestaltung der Erfindung ist das zumindest eine Betätigungselement ebenfalls rohrförmig ausgebildet.

Diese Maßnahme hat den Vorteil, daß das Betätigungselement, das im Innern des Instruments durchgeführt wird, den Hohlkanal ebenfalls geringstmöglich beeinträchtigt. Das Betätigungselement hat im wesentlichen Druck- oder Zugkräfte zu übertragen, so daß die Rohrgeometrie ideal ist, bei möglichst dünner Wandstärke hohe Kräfte zu übertragen. Das rohrförmige Betätigungselement kann so ausgebildet sein, daß dieses den Hohlraum umgrenzt.

In einer weiteren Ausgestaltung der Erfindung weist das Betätigungselement Steuerkurven darstellende Aussparungen auf, in die die Steuerzapfen der Arbeitselemente eingreifen.

Diese Maßnahme hat den Vorteil, daß die Wirkverbindung zwischen Betätigungselement und den Arbeitselementen durch Maßnahmen bewerkstelligt werden, die sich in Umfangsrichtung bzw. in Längsrichtung des Betätigungselements erstrecken können, somit der innere Hohlkanal durch diese Bauelemente nicht beeinträchtigt ist.

In einer weiteren Ausgestaltung der Erfindung wirkt das Betätigungselement über gelenkige Zwischenstücke auf die Arbeitselemente ein.

Diese Maßnahme hat ebenfalls den Vorteil, daß langerstreckte Lagen der gelenkigen Zwischenstücke möglich sind, die, sei es zum Einführen des Instrumentes selbst durch ein Trokar oder zum Einführen eines weiteren Instruments in den inneren Hohlraum, möglichst raumsparend angeordnet sind.

In einer weiteren Ausgestaltung der Erfindung wirkt das Betätigungselement über flexible Zwischenstücke auf die Arbeitselemente ein.

Auch diese Maßnahme hat den Vorteil, daß schlanke, die Bauweise nicht vergrößernde oder den inneren Hohlkanal nicht beeinträchtigende Bauelemente vorhanden sind.

In einer weiteren Ausgestaltung der Erfindung sind die Arbeitselemente über Drehgelenke am Schaft gelagert.

Diese Maßnahme hat den Vorteil, daß über die gelenkige Lagerung am Schaft eine ausreichende Stabilität der Arbeitselemente erzielt werden kann, wobei dies dennoch platzsparend durchzuführen ist.

In einer weiteren Ausgestaltung der Erfindung sind die Arbeitselemente über flexible Bänder oder Foliengelenke am Schaft gelagert.

Diese Maßnahme hat den Vorteil, daß flächige, in radialer Richtung schlank bauende Lagerungen möglich sind, die wiederum für die ausreichende Stabilität sorgen, weder die Baubreite vergrößern, noch den inneren Hohlkanal sperrend beeinträchtigen.

Die Lagerung wird in einer weiteren Ausgestaltung so gewählt, daß diese eine möglichst breite Basis hat, vorzugsweise nahezu den Außendurchmesser des Rohrschafts. Dies kann z.B. dadurch erreicht werden, daß die Basis jeweils als Halbring mit zwei diametral gegenüberliegenden Lagerungspunkten ausgebildet ist, an dem die Arbeitselemente bzw. Maulteile angesetzt sind.

In einer weiteren Ausgestaltung der Erfindung sind die Arbeitselemente als zwei Maulteile einer Schere oder einer Faßzange ausgebildet.

Diese Maßnahme hat den Vorteil, daß das chirurgische Instrument als ein häufig eingesetztes Instrument ausgebildet ist, bei dem bei den Faß- bzw. Schneidevorgängen auch sehr starke Kräfte übertragen werden müssen, die aufgrund der zuvor genannten Ausgestaltungen problemlos in schlanker Bauweise unter Freilassen eines großen Hohlkanals durchzuführen sind.

In einer weiteren Ausgestaltung der Erfindung sind die Maulteile als sich umfänglich etwa über 90° erstreckende, nebeneinander liegende Rohrabschnitte ausgebildet, deren gegenüberstehende Kanten scherenartig aneinander vorbeilaufen.

Diese bevorzugte Ausgestaltung hat den erheblichen Vorteil, daß mit dieser Ausgestaltung die Maulteile weder die Baubreite des Schaftes über dessen äußeren Umfang hinaus verbreitern, noch der innere Hohlkanal beeinträchtigt ist, so daß bei minimalem Schaftdurchmesser ein maximaler innerer Hohlkanal zur Verfügung steht, der vollkommen durchgängig ist, so daß auch die Instrumente über die Maulteile hinausgeschoben werden können, unabhängig davon, ob diese geschlossen oder gespreizt sind.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels näher beschrieben und erläutert. Es zeigen:
- Fig. 1: eine Seitenansicht eines erfindungsgemäßen chirurgischen Instruments, das als Schere ausgebildet ist, in das ein weiteres Instrument, nämlich ein Endoskop eingeschoben ist,
- Fig. 2: eine perspektivische Ansicht des distalen Endes des chirurgischen Instruments,
- Fig. 3: einen Schnitt längs der Linie III-III in Fig. 2 bzw. Fig. 4,
- Fig. 4: eine teilweise längsgeschnittene Seitenansicht des distalen Endbereichs des chirurgischen Instruments von Fig. 1,
- Fig. 5: eine gegenüber der Darstellung von Fig. 4 um 90° um die Schaftachse gedrehte Draufsicht auf den distalen Endbereich bei geschlossenen Maulteilen, und
- Fig. 6: eine der Fig. 5 vergleichbare Darstellung mit geöffneten bzw. gespreizten Maulteilen.

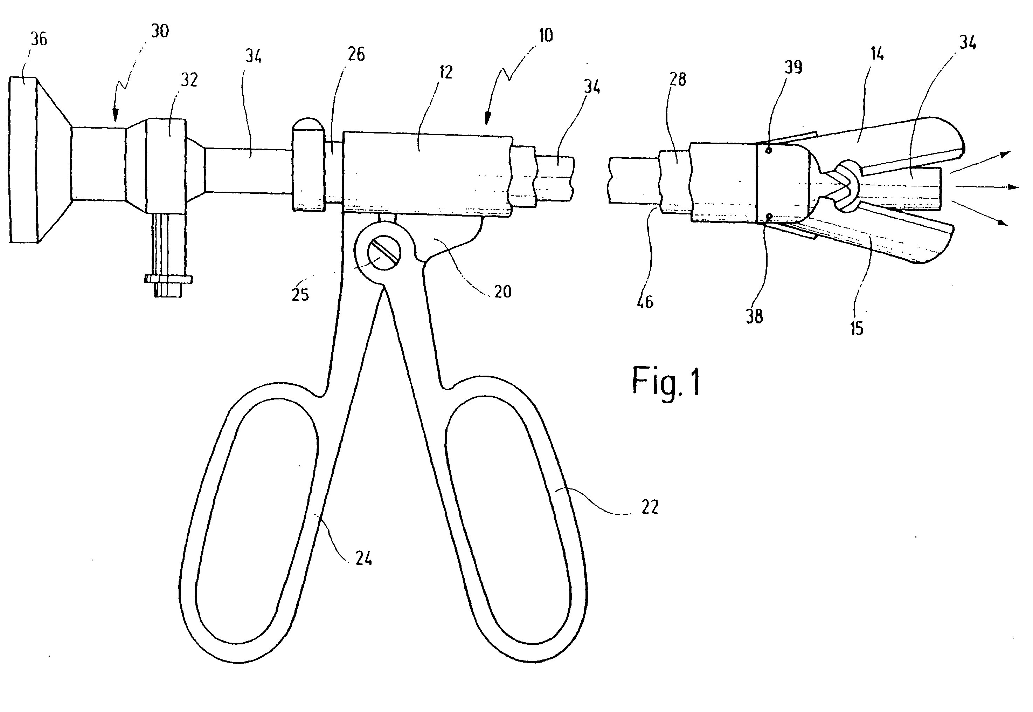

In Fig. 1 ist ein chirurgisches Instrument in seiner Gesamtheit mit der Bezugsziffer 10 bezeichnet.

Das chirurgische Instrument 10 ist eine Schere, die einen mittigen längserstreckten rohrförmigen Schaft 12 aufweist.

Am distalen Ende des Schaftes 12 sind zwei Arbeitselemente 14, 15 angelenkt, die als Maulteile 16 und 17 ausgestaltet sind, wie das später noch näher beschrieben wird. Am proximalen Ende des Schaftes 12 ist ein Griff 20 angeordnet, der zwei Griffteile 22 und 24 aufweist, die über ein Scharnier 25 miteinander verbunden sind.

Das Griffteil 24 ist fest mit der Außenseite des proximalen Endes des Schaftes 12 verbunden, das Griffteil 22 ist das bewegliche Griffteil. Das bewegliche Griffteil 22 ist mit einem sich im Innern des Schaftes 12 erstreckenden Betätigungselement 26 verbunden, das die Form eines Rohres 28 aufweist. Der Außendurchmesser des Rohres 28 entspricht dabei dem lichten Innendurchmesser des rohrförmigen Schaftes 12. Durch Bewegen des beweglichen Griffteils 22 kann das Betätigungselement 26 im Schaft hin- und herverschoben werden.

Es ist auch möglich, daß das bewegliche Griffteil 22 fest mit dem Schaft 12 verbunden wird, und daß das unbewegliche Griffelement 24 durch einen Schlitz im Schaft 12 durchreichend fest mit dem Betätigungselement 26 verbunden wird. Dann wird der Schaft 12 verschoben. Diese kinematischen Varianten werden in Abhängigkeit davon gewählt, ob beim Spreizen oder beim Schließen der Arbeitselemente 14, 15 die maximale und für die Handhabungsperson am einfachsten aufzubringende Kraft ausgeübt werden soll. Am einfachsten ist dies für die Handhabungsperson so durchzuführen, daß die Griffteile 22 und 24 von Hand ergriffen und die Griffteile 22 und 24 aufeinander zu bewegt werden. Bei Faßzangen und Scheren spielt vorrangig die Schließkraft eine Rolle, bei Spreizern die Spreizkraft.

Das distale Ende des Betätigungselements 26 steht über später noch zu beschreibende Achsgelenke mit den Arbeitselementen 14, 15 in Verbindung, wobei in Fig. 1 die Schwenkachsen 38 und 39 zu erkennen sind, um die die Arbeitselemente 14, 15 verschwenkt werden, wenn das Betätigungselement 26 hin- und hergeschoben wird.

Im in Fig. 1 dargestellten Zustand sind die Arbeitselemente 14, 15 gespreizt.

Das chirurgische Instrument 10 ist nun so ausgebildet, daß von dem proximalen Ende her ein weiteres rohrförmiges Instrument 30, im dargestellten Ausführungsbeispiel ein Endoskop 32 eingeschoben werden kann.

Der Außendurchmesser des Schaftes 34 des Endoskopes 32 entspricht dabei etwa dem lichten Innendurchmesser des Rohres 28, das das Betätigungselement 26 darstellt. In Fig. 1 ist dargestellt, daß der Schaft 34 des Endoskopes 32 so weit durch das Instrument 10 hindurchgeschoben ist, daß dessen distales Ende bis nahezu an die äußeren distalen Enden der Arbeitselemente 14, 15 reicht. Diese Stellung kann bspw. dann gewählt werden, wenn das chirurgische Instrument 10 bzw. dessen Arbeitselemente 14, 15 an Ort und Stelle gebracht werden sollen, dies kann dann über die Okularmuschel 36 des Endoskopes 32 visuell verfolgt werden. Soll zwischen den scherenförmigen Maulteilen 16 und 17 ein Gewebe erfaßt und durchgetrennt werden, wird das Endoskop 32 entsprechend weit zurückgezogen.

Im dargestellten Ausführungsbeispiel ist ein Endoskop als weiteres Instrument 30 in das chirurgische Instrument 10 eingeschoben, es können selbstverständlich auch andere entsprechend ausgestaltete Instrumente eingeschoben werden.

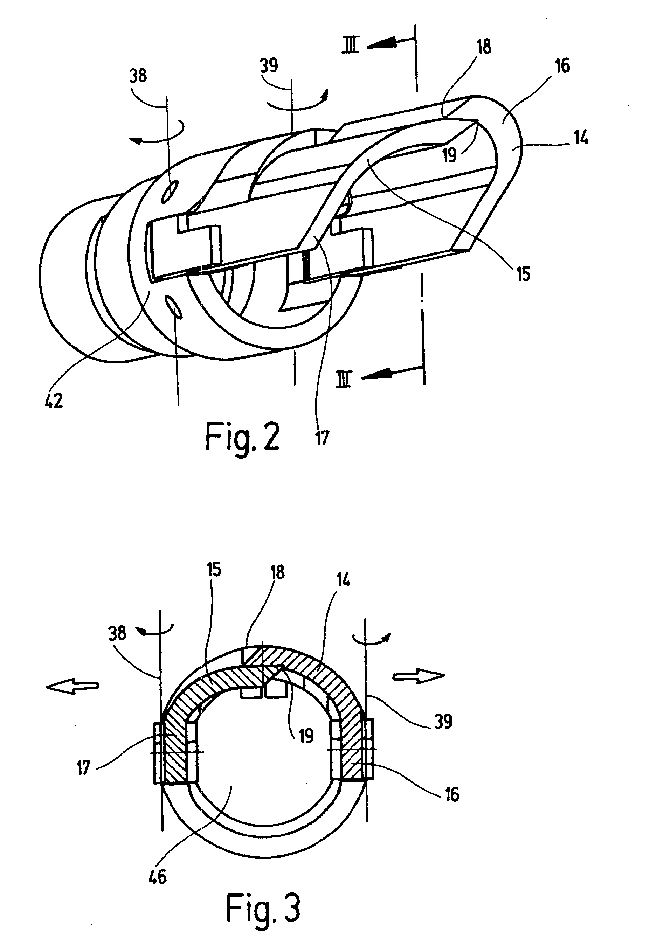

In Zusammenhang mit den Fig. 2 bis 6 soll die nähere Ausgestaltung der Arbeitselemente 14 und 15 als scherenartige Maulteile 16 und 17 und deren Steuerung näher beschrieben werden.

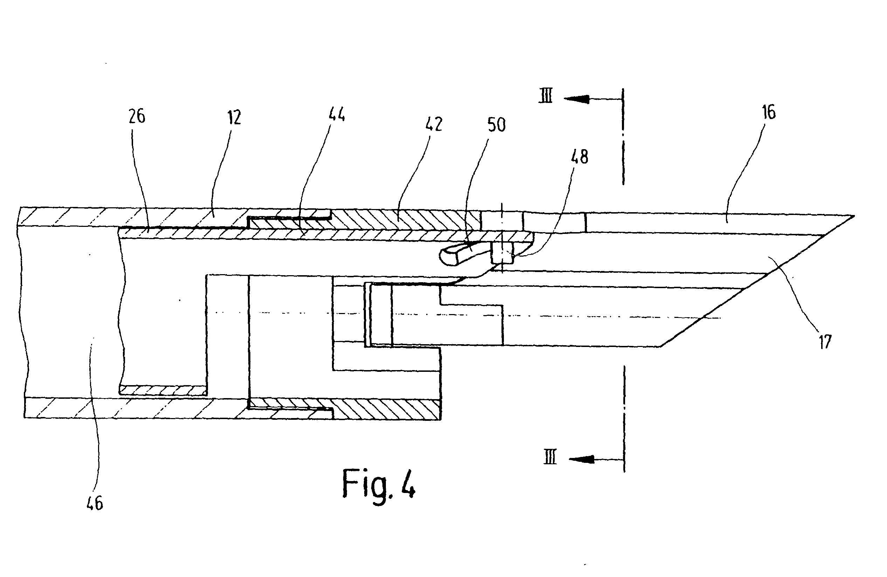

In Fig. 2 ist ein Rohrstück 42 dargestellt, an dem die Maulteile 16 und 17 angebracht sind, das dann vom distalen Ende her in den Schaft 12 eingeschoben und mit diesem fest verbunden wird, wie das aus der Schnittdarstellung von Fig. 4 ersichtlich ist.

Dies erleichtert die Montage und Fertigung dieser Bauteile.

Aus den Darstellungen von Fig. 2 und 3 ist zu entnehmen, daß die Maulteile 16 und 17 als etwa rohrabschnittförmige Teile ausgebildet sind, die über sich quer zur Längsachse des Schaftes 12 erstreckende Achszapfen (die hier nicht dargestellt sind) mit dem Rohrstück 42 verbunden sind.

Die Mittellängsachsen dieser Achszapfen sind dann die Dreh- bzw. Schwenkachsen 38 und 39 der Maulteile 16 und 17.

Die sich gegenüberstehenden Kanten 18 und 19 der Maulteile 16 und 17 sind so ausgebildet, daß sie scherenartig aneinander vorbei gleiten können, wodurch dann ein atraumatischer exakter Schnitt durchgeführt werden kann.

Dazu sind die Kanten 18 und 19 angeschrägt, wie das insbesondere aus der Schnittdarstellung von Fig. 3 zu erkennen ist.

Das Maulteil 16 entspricht dabei einem sich etwas umfänglich über 90° hinaus erstreckenden Rohrabschnitt, der sich in etwa in Verlängerung der rohrförmigen Wand des Schaftes 12 erstreckt.

Das andere Maulteil 17 ist im Bereich dessen Kante so radial nach innen gebogen, daß es unter das andere Maulteil, also radial weiter innenliegend bewegt werden kann. Bei der Spreizbewegung bewegen sich die Maulteile 16 und 17, wie in Fig. 3 durch Pfeile angedeutet, voneinander weg, bei der entgegengesetzten Bewegung schließen sie sich wieder in die in Fig. 2 und 3 dargestellte Schließstellung. Dabei laufen die Kanten 18 und 19 scherenartig aneinander vorbei.

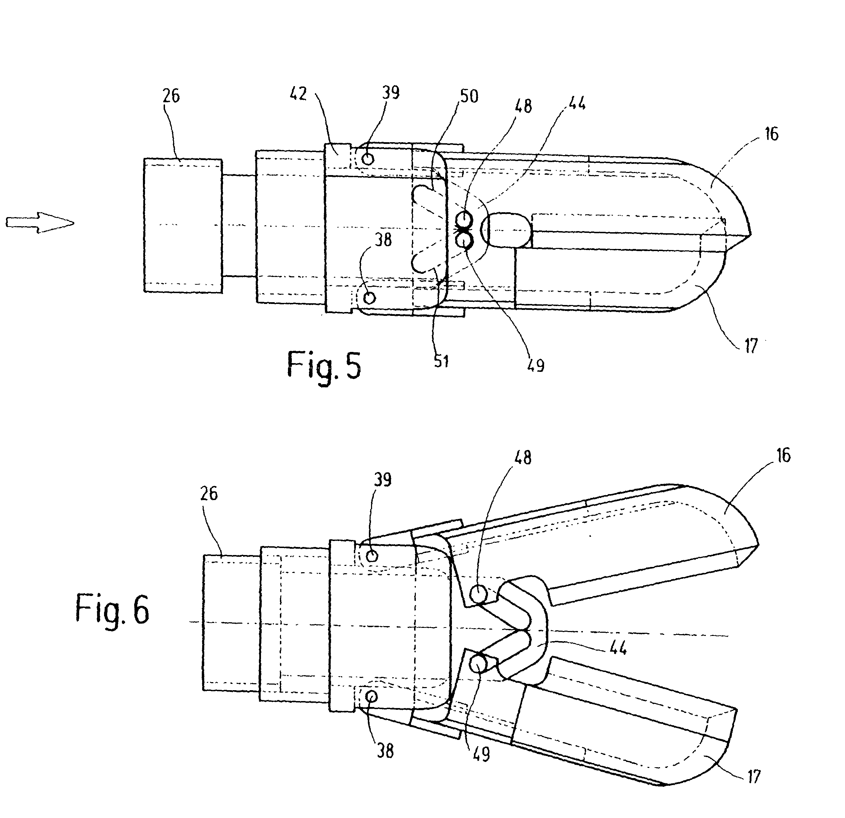

Die Steuerung der Spreizbewegung ist insbesondere aus Fig. 5 und 6 ersichtlich.

Daraus ist zu erkennen, daß an einem distalen Fortsatz 44 des Rohres 28 des Betätigungselements 26 als Steuerkurven 50 und 51 dienende Aussparungen vorgesehen sind, in die entsprechende Steuerzapfen 48 und 49 eingreifen, die radial nach innen von der Innenseite der Maulteile 16 und 17 vorspringen.

Die Steuerkurven 50 und 51 sind als Langlöcher ausgebildet, die sich zum distalen Ende hin V-förmig aufeinander zu bewegen.

Wird das Betätigungselement 26 bei geschlossener Stellung der Maulteile 16 und 17, wie sie in Fig. 5 dargestellt ist, nach distal verschoben, wie das durch einen Pfeil angedeutet ist, so werden die Steuerzapfen 48 und 49 durch die Steuerkurven 50 und 51 radial nach außen bewegt, wodurch die Maulteile 16 und 17 gespreizt werden, diese verschwenken dabei um die Achsen 38 und 39.

Wird das Betätigungselement 26 nach proximal bewegt, findet dann der umgekehrte Vorgang statt, d.h. die Maulteile 16 und 17 schließen wieder.

Diese Linearbewegung wird über das bewegliche Griffteil 22 bewirkt, das dazu mit dem Betätigungselement 26 in Wirkverbindung steht. Auch hier ist wieder eine kinematische Umkehr möglich, also das bewegliche Griffteil ist mit dem Schaft verbunden und das unbewegliche mit dem Betätigungselement.

Insbesondere aus der Schnittdarstellung von Fig. 3 ist zu erkennen, daß durch diese Konstruktion ein relativ großer innerer Hohlkanal 46 vorhanden ist, der weder durch sperrige Bauelemente beeinträchtigt ist, noch bei der Schließ- bzw. Öffnungsbewegung beeinträchtigt wird, da die Steuermechanismen bzw. Anlenkungspunkte jeweils umfänglich verteilt sind. Somit kann in das chirurgische Instrument 10 ein weiteres Instrument 30 eingeschoben werden, das nur einen unwesentlich geringeren Außendurchmesser aufweist. Insgesamt gesehen, wie das aus Fig. 1 ersichtlich ist, benötigt der Zusammenbau aus chirurgischem Instrument 10 und darin eingeschobenem weiteren Instrument 30 nur eine äußerst geringe radiale Baugröße, so daß beide Bauelemente über eine einzige kleine Inzision bzw. einen entsprechenden Trokar in einen Körper eingeführt bzw. von diesem abgezogen werden können.

## Patentansprüche

1. Chirurgisches Instrument, mit einem rohrförmigen Schaft (12), an dessen distalem Ende Arbeitselemente (14, 15) angeordnet sind, von denen zumindest eines spreizbar ist, wobei die Arbeitselemente (14, 15) über ein Betätigungselement (26) mit einem am proximalen Ende des Schaftes (12) angeordneten Griff (20) zum Öffnen und Schließen der Arbeitselemente (14, 15) in Wirkverbindung stehen, und mit einem im Innern des Schaftes (12) durchgehenden Hohlkanal (46), durch welchen Hohlkanal (46) zumindest ein weiteres Instrument einführbar ist, wobei entweder der Durchmesser des Hohlkanals dem lichten Innendurchmesser des Schaftes (12) entspricht, oder das Betätigungselement (26) als ein im Schaft angeordnetes Rohr (28) ausgebildet ist und der Durchmesser des Hohlkanals dem lichten Innendurchmesser des Rohres (28) entspricht, **dadurch gekennzeichnet, dass** die Arbeitselemente (14, 15) derart ausgestaltet sind, dass sie weder im geschlossenen noch im gespreiztem Zustand in einen Raum hineinreichen, der durch eine gedachte Verlängerung des Hohlkanales (46) an dessen distalem Ende gebildet wird.

2. Chirurgisches Instrument nach Anspruch 1, **dadurch gekennzeichnet, daß** die am distalen Ende angeordneten Arbeitselemente (14, 15) in einer Arbeitsstellung sich etwa in einer Verlängerung der Rohrwand des rohrförmigen Schaftes (12) erstrecken.

3. Chirurgisches Instrument nach Anspruch 1 oder 2, **dadurch gekennzeichnet, daß** die Arbeitselemente (14, 15) selbst rohr- bzw. rohrabschnittförmig ausgebildet sind.

4. Chirurgisches Instrument nach einem der Ansprüche 1 bis 3, **dadurch gekennzeichnet, daß** das Betätigungselement (26) ebenfalls rohrförmig ausgebildet ist.

5. Chirurgisches Instrument nach einem der Ansprüche 1 bis 4, **dadurch gekennzeichnet, daß** das Betätigungselement (26) Steuerkurven (50, 51) darstellende Aussparungen aufweist, in die Steuerzapfen (48, 49) der Arbeitselemente (14, 15) eingreifen.

6. Chirurgisches Instrument nach einem der Ansprüche 1 bis 4, **dadurch gekennzeichnet, daß** das Betätigungselement über gelenkige Zwischenstücke auf die Arbeitselemente (14, 15) einwirkt.

7. Chirurgisches Instrument nach einem der Ansprüche 1 bis 4, **dadurch gekennzeichnet, daß** das Betätigungselement über flexible Zwischenstücke auf die Arbeitselemente (14, 15) einwirkt.

8. Chirurgisches Instrument nach einem der Ansprüche 1 bis 7, **dadurch gekennzeichnet, daß** die Arbeitselemente (14, 15) über Drehgelenke (38, 39) am Schaft (12) gelagert sind.

9. Chirurgisches Instrument nach einem der Ansprüche 1 bis 7, **dadurch gekennzeichnet, daß** die Arbeitselemente (14, 15) über flexible Bänder oder Foliengelenke am Schaft (12) gelagert sind.

10. Chirurgisches Instrument nach einem der Ansprüche 1 bis 9, **dadurch gekennzeichnet, daß** Lagerstellen der Arbeitselemente umfänglich möglichst weit voneinander beabstandet sind.

11. Chirurgisches Instrument nach einem der Ansprüche 1 bis 10, **dadurch gekennzeichnet, daß** die Arbeitselemente (14, 15) als Maulteile (16, 17) einer Schere oder einer Faßzange ausgebildet sind.

12. Chirurgisches Instrument nach Anspruch 11, **dadurch gekennzeichnet, daß** die Maulteile (16, 17) als sich umfänglich etwa über 90° erstreckende, nebeneinanderliegende Rohrabschnitte ausgebildet sind, deren gegenüberstehende Kanten (18, 19) scherenartig aneinander vorbei laufen.

## Claims

1. Surgical instrument comprising a tubular shaft (12) having working elements (14, 15) arranged at its distal end, at least one of which may be spread apart, wherein the working elements (14, 15) are operatively connected via an actuator element (26) with a handle (20) arranged at the proximal end of the shaft (12) for opening and closing the working elements (14, 15) and a continuous hollow channel in the interior of the shaft (12) through which hollow channel (46) at least a further instrument can be introduced, wherein either the diameter of the hollow channel corresponds to the inner clearance diameter of the tubular shaft (12), or the actuator element (26) is formed as a tube (28) disposed within the shaft and the diameter of the hollow channel (46) corresponds to the inner clearance diameter of the tube (28), **characterized in that** the working elements (14, 15) are configured such that they do not extend into a space neither in opened nor in closed condition which space is formed by a virtual prolongation of the hollow channel (46) at its distal end.

2. Surgical instrument of claim 1, **characterized in that** the working elements (14, 15) arranged at the distal end project in a working position as an extension of the tube wall of the tubular shaft (12).

3. Surgical instrument of claims 1 or 2, **characterized in that** the working elements (14, 15) themselves are formed as tubes or tube sections.

4. Surgical instrument of anyone of claims 1 through 3, **characterized in that** the actuator element (26) is also formed to be tubular.

5. Surgical instrument of anyone of claims 1 through 4, **characterized in that** the actuator element (26) comprises recesses acting as guide slots (50, 51) into which guide pins (48, 49) of the working elements (14, 15) engage.

6. Surgical instrument of anyone of claims 1 through 4, **characterized in that** the actuator element engages with the working elements (14, 15) through pivotal intermediate means.

7. Surgical instrument of anyone of claims 1 through 4, **characterized in that** the actuator element engages the working elements (14, 15) through flexible intermediate means.

8. Surgical instrument of anyone of claims 1 through 7, **characterized in that** the working elements (14, 15) are mounted on the shaft (12) through pivotal joints (38, 39).

9. Surgical instrument of anyone of claims 1 through 7, **characterized in that** the working elements (14, 15) are mounted on the shaft (12) through flexible band or foil joints.

10. Surgical instrument of anyone of claims 1 through 9, **characterized in that** mounting points of the working elements are spaced as far as possible from another in circumferential direction.

11. Surgical instrument of anyone of claims 1 through 10, **characterized in that** the working elements (14, 15) are configured as jaws (16, 17) of a scissors or a grasping forceps.

12. Surgical instrument of claim 1, **characterized in that** the jaws (16, 17) are formed as adjacent tube sections extending about 90° in the circumferential direction, whose opposing ends (18, 19) pass by one another in scissor-like manner.

## Revendications

1. Instrument chirurgical, avec une tige tubulaire (12) à l'extrémité distale de laquelle sont agencés des éléments de travail (14, 15) dont au moins un est expansible, les éléments de travail (14, 15) étant en liaison active avec une poignée (20) placée sur l'extrémité proximale de la tige (12), par l'intermédiaire d'un élément d'actionnement (26), pour ouvrir et fermer les éléments de travail (14, 15), et avec un conduit creux (46) traversant à l'intérieur de la tige (12), canal creux (46) à travers lequel on peut introduire au moins un autre instrument, sachant que soit le diamètre du conduit creux correspond au diamètre intérieur de la tige (12) soit l'élément d'actionnement (26) est configuré comme un tube (28) placé dans la tige, et le diamètre du conduit creux correspond au diamètre intérieur du tube (28), **caractérisé en ce que** les éléments de travail (14, 15) sont conçus de telle manière que, ni à l'état fermé ni à l'état expansé, ils ne pénètrent jusque dans un espace qui est formé par un prolongement imaginaire du conduit creux (46) à l'extrémité distale du celui-ci.

2. Instrument chirurgical selon la revendication 1, **caractérisé en ce que** les éléments de travail (14, 15) agencés à l'extrémité distale s'étendent, dans une position de travail, à peu près dans un prolongement de la paroi du tube de la tige tubulaire (12).

3. Instrument chirurgical selon la revendication 1 ou 2, **caractérisé en ce que** les éléments de travail (14, 15) sont configurés eux-mêmes en forme de tube ou de tronçon de tube.

4. Instrument chirurgical selon l'une quelconque des revendications 1 à 3, **caractérisé en ce que** l'élément d'actionnement (26) est également configuré en forme de tube.

5. Instrument chirurgical selon l'une quelconque des revendications 1 à 4, **caractérisé en ce que** l'élément d'actionnement (26) comprend des courbes des cavités décrivant des cames de commande (50, 51) dans lesquelles s'engrènent des tourillons de commande (48, 49) des éléments de travail (14, 15).

6. Instrument chirurgical selon l'une quelconque des revendications 1 à 4, **caractérisé en ce que** l'élément d'actionnement agit sur les éléments de travail (14, 15) par l'intermédiaire de pièces intercalaires articulées.

7. Instrument chirurgical selon l'une quelconque des revendications 1 à 4, **caractérisé en ce que** l'élément d'actionnement agit sur les éléments de travail (14, 15) par l'intermédiaire de pièces intercalaires flexibles.

8. Instrument chirurgical selon l'une quelconque des revendications 1 à 7, **caractérisé en ce que** les éléments de travail (14, 15) sont logés sur la tige (12) à l'aide d'articulations tournantes (38, 39).

9. Instrument chirurgical selon l'une quelconque des revendications 1 à 7, **caractérisé en ce que** les éléments de travail (14, 15) sont logés sur la tige (12) à l'aide de bandes flexibles ou d'articulations à lamelles.

10. Instrument chirurgical selon l'une quelconque des revendications 1 à 9, **caractérisé en ce que** les points d'appui des éléments de travail (14, 15) sur la circonférence sont le plus éloigné possible les uns des autres.

11. instrument chirurgical selon l'une quelconque des revendications 1 à 10, **caractérisé en ce que** les éléments de travail (14, 15) sont configurés comme des parties de bouche (16, 17) d'une cisaille ou d'une tenaille de pinçage.

12. instrument chirurgical selon la revendication 11, **caractérisé en ce que** les parties de bouche (16, 17) sont configurées comme des tronçons de tube placés les uns à côté des autres, s'étendant sur environ 90° en périphérie, dont les arêtes opposées (18, 19) passent l'une devant l'autre à la manière d'une cisaille.
